# EUROPEAN PATENT SPECIFICATION

(11) **EP 4 182 484 B1**
(45) Date of publication and mention of the grant of the patent: **27.05.2026**
(21) Application number: 21843383.7
(22) Date of filing: 14.07.2021
(51) Int. Cl.: C12Q 1/70, A61B 10/00

(54) **SAMPLING KITS AND METHODS FOR DETECTING RESPIRATORY PATHOGENS**
PROBENAHMEKITS UND VERFAHREN ZUM NACHWEIS VON ERREGERN DER ATEMWEGE
KITS DE PRÉLÈVEMENT ET MÉTHODES DE DÉTECTION D'AGENTS PATHOGÈNES RESPIRATOIRES

(30) Priority: 14.07.2020 KR 20200087107
(43) Date of publication of application: 24.05.2023
(73) Proprietor: Seegene, Inc., Seoul 05548 (KR)
(72) Inventor: CHUN, Jong Yoon, Seoul 06075 (KR)
(74) Representative: Kilger, Ute
(86) International application number: PCT/KR2021/009055
(87) International publication number: WO 2022/015053

(56) References cited:
- WO-A1-2022/171967
- WO-A1-2022/192440
- WO-A1-2023/287901
- US-A1- 2014 127 671
- US-A1- 2017 227 548
- ROBINSON JOAN L., LEE BONITA E., KOTHAPALLI SUSHMA, CRAIG WILLIAM R., FOX JULIE D.: "Use of Throat Swab or Saliva Specimens for Detection of Respiratory Viruses in Children", CLINICAL INFECTIOUS DISEASES, THE UNIVERSITY OF CHICAGO PRESS, CHICAGO, IL., US, vol. 46, no. 7, 1 April 2008 (2008-04-01), US , pages e61 - e64, XP055887112, ISSN: 1058-4838, DOI: 10.1086/529386
- IWASAKI SUMIO; FUJISAWA SHINICHI; NAKAKUBO SHO; KAMADA KEISUKE; YAMASHITA YU; FUKUMOTO TATSUYA; SATO KAORI; OGURI SATOSHI; TAKI KE: "Comparison of SARS-CoV-2 detection in nasopharyngeal swab and saliva", JOURNAL OF INFECTION., ACADEMIC PRESS, LONDON., GB, vol. 81, no. 2, 4 June 2020 (2020-06-04), GB , XP086228485, ISSN: 0163-4453, DOI: 10.1016/j.jinf.2020.05.071
- M.K. AKMATOV; F. PESSLER;: "Self-collected nasal swabs to detect infection and colonization: a useful tool for population-based epidemiological studies?", INTERNATIONAL JOURNAL OF INFECTIOUS DISEASES, INTERNATIONAL SOCIETY FOR INFECTIOUS DISEASES, HAMILTON, CA, vol. 15, no. 9, 22 April 2011 (2011-04-22), CA , pages e589 - e593, XP028269923, ISSN: 1201-9712, DOI: 10.1016/j.ijid.2011.04.009
- KIM YOUNG-GON, YUN SEUNG GYU, KIM MIN YOUNG, PARK KWISUNG, CHO CHI HYUN, YOON SOO YOUNG, NAM MYUNG HYUN, LEE CHANG KYU, CHO YUN-JU: "Comparison between Saliva and Nasopharyngeal Swab Specimens for Detection of Respiratory Viruses by Multiplex Reverse Transcription-PCR", JOURNAL OF CLINICAL MICROBIOLOGY, AMERICAN SOCIETY FOR MICROBIOLOGY, US, vol. 55, no. 1, 1 January 2017 (2017-01-01), US , pages 226 - 233, XP055887114, ISSN: 0095-1137, DOI: 10.1128/JCM.01704-16

## Description

### BACKGROUND OF THE INVENTION

### FIELD OF THE INVENTION

The present invention relates to sampling kits and methods for detecting respiratory pathogens.

### DESCRIPTION OF THE RELATED ART

Respiratory diseases cause serious mortality, especially in children, the elderly, and immunocompromised people. Identifying causative pathogens is very important for infection control and appropriate patient care. Respiratory pathogen testing has developed rapidly with the development of molecular diagnostic technology, especially real-time PCR. Real-time PCR is very sensitive and prompt, and can simultaneously detect various pathogens compared with conventional methods.

Various upper respiratory tract (URT) samples and lower respiratory tract samples have been used for respiratory pathogen testing by molecular diagnosis.

In general, nasopharyngeal swab samples and oropharyngeal swab samples are widely used as upper respiratory samples. Sputum and bronchoalveolar lavage (BAL) samples are used as lower respiratory samples.

In recent years, real-time PCR diagnostic kits are greatly spotlighted due to the SARS-CoV-2 pandemic. For coronavirus diagnosis, nasopharyngeal swab samples and oropharyngeal swab samples are used as upper respiratory tract samples, and sputum and bronchoalveolar lavage are used as lower respiratory tract samples. The nasopharyngeal and oropharyngeal swab samples are obtained by collecting secretions from the nasopharynx and oropharynx using cotton swabs and then stored in transport media. The sputum is collected and stored in a sterile vessel after the mouth is rinsed with saline.

However, lower respiratory tract samples are difficult to collect and may require pretreatment before nucleic acid extraction, and especially, sputum samples cannot be applied in the early stages of infection in which patients have substantially no cough symptoms or a dry cough. Sampling by nasopharyngeal and oropharyngeal swabs has its limitations that need to be conducted by an expert and is risky due to its aerosol-generating sampling manner.

Under the above-described circumstances in the art, there is a need in the art for a respiratory pathogen testing method in which a novel sampling method is introduced.

US2017/227548 A1 discloses detecting levels of a biomarker in a biological sample which may comprise nasal mucus, saliva or a combination thereof.

### SUMMARY OF THE INVENTION

The present inventors have made intensive researches to develop a novel protocol for a detection method capable of detecting respiratory pathogens. As a result, the present inventors completed a new protocol for detection of respiratory pathogens, in which a nostril swab sample and a saliva sample are obtained, both of these are impregnated in a sample transport medium, and then a nucleic acid amplification reaction is performed using a nucleic acid molecule obtained from the sample transport medium. The method of the present invention has advantages of showing a positive rate equivalent to that in a conventional detection method using nasopharyngeal swabs and allowing self-sampling.

Accordingly, it is an object of the present invention to provide a method for detecting respiratory pathogens.

It is another object of the present invention to provide the use of a sampling kit for detecting respiratory pathogens.

It is still another object of the present invention to provide the use of a kit for detecting respiratory pathogens.

It is another object of the present invention to provide a sampling method for detecting respiratory pathogens.

Other objects and advantages of the present invention will become apparent from the detailed description to follow taken in conjugation with the appended claims and drawings.

### BRIEF DESCRIPTION OF THE DRAWINGS

FIG. 1 illustrates one embodiment of a nostril swab and/or a saliva swab used in the present invention.
FIG. 2A illustrates one embodiment of how to collect a sample by using a nostril swab.
FIG. 2B illustrates one embodiment of how to collect a sample by using a saliva swab.

### DETAILED DESCRIPTION OF THIS INVETNION

In an aspect of the present invention, there is provided a method for detecting a respiratory pathogen, the method including:
(a) performing a nucleic acid amplification reaction by using a sample transport medium in which a nostril swab sample and a saliva sample are dipped together and a nucleic acid amplification reaction composition; and
(b) analyzing the results of the nucleic acid amplification reaction to determine the presence or absence of the respiratory pathogen.

The present inventors have made intensive researches to develop a detection method as a novel protocol capable of detecting respiratory pathogens. As a result, the present inventors completed a new protocol for detection of respiratory pathogens, in which a nostril swab sample and a saliva sample are obtained, both of these are impregnated with a sample transport medium, and then a nucleic acid amplification reaction is performed using a nucleic acid molecule obtained from the sample transport medium. The method of the present invention has advantages of showing a positive rate equivalent to that in a conventional detection method using nasopharyngeal swabs and allowing self-sampling.

The detection method of the present invention will be described in detail according to each step as follows:

### Step (a): Nucleic acid amplification reaction using nostril swab sample and saliva sample

A nucleic acid amplification reaction is performed using a sample transport medium in which a nostril swab sample and a saliva sample are dipped together and a nucleic acid amplification reaction composition.

The samples used in the present invention are a nostril swab sample and a saliva sample.

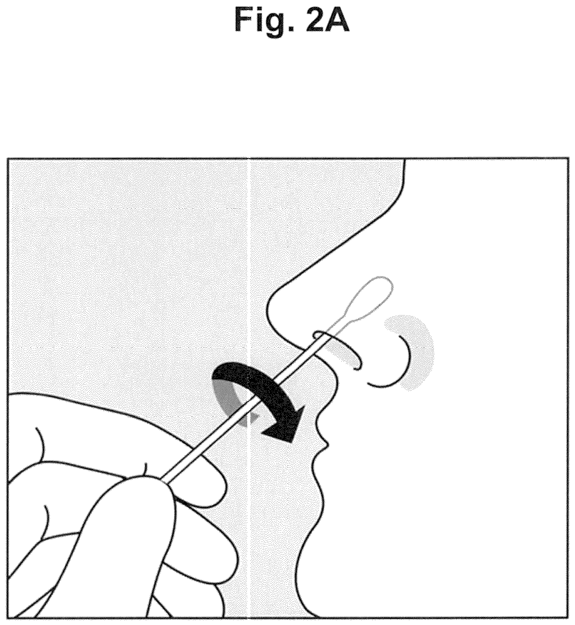

As used herein, the term "nostril swab sample" refers to a swab sample, which is obtained by applying a swab device to nostrils and onto which a biopsy sample from the nostrils is adsorbed, and the term is distinguished from a nasopharyngeal swab sample. For example, referring to FIG. 2A, a swab device is inserted into a nostril (e.g., insert 2-3 cm), and then a biopsy sample is taken from the inner portion of the nostril while the swab is smoothly rotated for 10-15 seconds. The term "nostril swab sample" is used interchangeably with "a nasal swab sample" so long as the nasal swab sample is also obtained by applying a swab device to nostrils as described above.

According to an embodiment of the present invention, the nostril swab sample is obtained by applying one swab device to both of two nostrils.

The saliva sample used in the present invention may include a sample obtained through the act of spitting (spitted saliva sample) and a sample obtained by applying a saliva swab device.

The spitted saliva sample obtained through the act of spitting may be obtained by spitting saliva directly into a container containing a sample transport medium or spitting saliva through a funnel into a container containing a sample transport medium.

According to an embodiment, the saliva sample is a saliva swab sample.

As used herein, the term "saliva swab sample" refers to a sample obtained by collecting saliva using a swab device.

According to an embodiment of the preset invention, the saliva swab sample is obtained by applying a swab device to a supralingual part or a sublingual part.

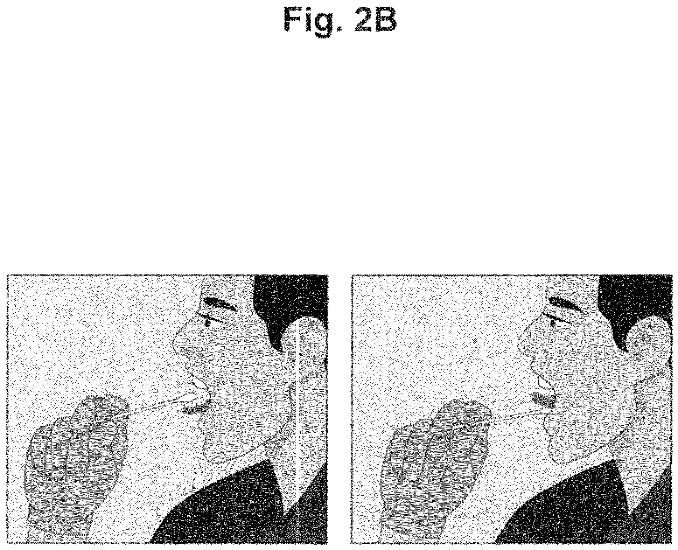

For example, the saliva swab sample may be obtained by applying a swab device to the surface of the tongue to allow saliva to be sufficiently absorbed into a fibrous layer of the swab device; or collecting saliva from the end of the tongue and then allowing the saliva to be sufficiently absorbed into the fibrous layer of the swab device (see FIG. 2B)
Alternatively, the saliva swab sample may be obtained by allowing saliva to sufficiently be absorbed while swabbing the sublingual part (see FIG. 2B).

According to an embodiment, the saliva swab sample is obtained by applying a swab device to a sublingual part.

According to an embodiment, the saliva swab sample may be obtained by applying a swab device to the inside of cheek, the gingiva and/or palate.

The term "saliva swab sample" is used interchangeably with an oral swab sample or oral saliva swab sample so long as the oral swab sample or oral saliva swab sample is also obtained by applying a swab device to a suitable part described above in order to mainly collect saliva.

According to an embodiment of the preset invention, the nostril swab sample and the saliva swab sample are obtained by applying two swab devices to the nostrils and tongue, respectively. Alternatively, the nostril swab sample and the saliva swab sample are obtained by applying one swab device to the nostrils and then the tongue.

According to an embodiment, the nostril swab sample and the saliva sample are obtained through self-sampling. The nostril swab sample and the saliva sample may be easily obtained by an ordinary person even without the involvement of experts, and thus a valid sample necessary for molecular diagnosis may also be obtained by self-sampling.

The entire shape of the swab device used in the present invention may be a shape known in the art (see U.S. Patent No. 10327741). For example, the swab may have a support part and a head part with a fibrous layer.

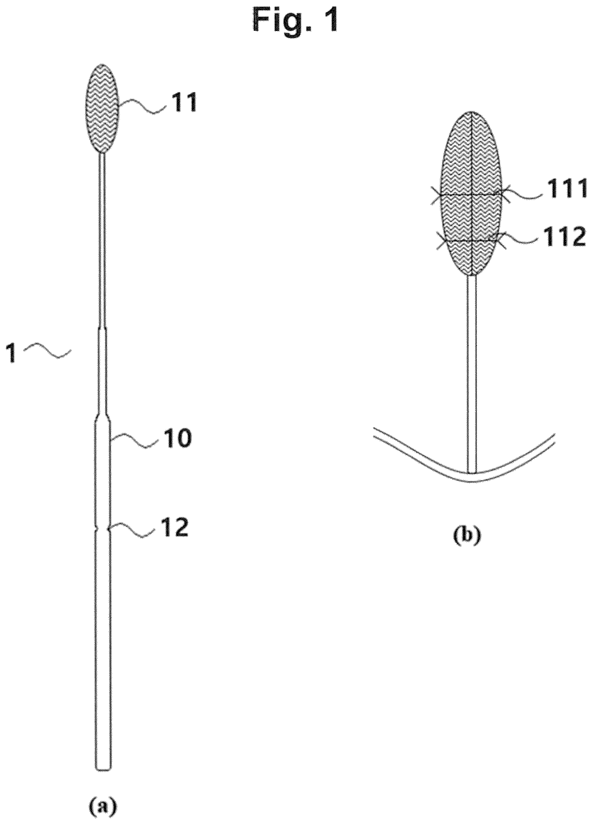

FIG. 1 illustrates a nostril swab and/or saliva swab usable in the present invention. A swab 1 includes a support part 10 and a head part 11 with a fibrous layer. The head part 11, together with the fibrous layer, may have an elliptical shape. The swab 1 may include a breaking point 12.

More specifically, the head part of the nostril swab and/or saliva swab is elliptical. The fibrous layer with an elliptical shape contains a horizontal long axis portion 111 and a horizontal short axis portion 112.

As used while describing the head part of the swab, the term "horizontal long axis portion" refers to a portion representing the longest axial length among axes perpendicular to the longitudinal axis of the length direction of the swab in the fibrous layer having an elliptical shape and the term "horizontal short axis portion" refers to an axis portion other than a portion indicating the longest axial length among axes perpendicular to the longitudinal axis.

Since the fibrous layer of the swab has an elliptical shape, the axis representing the longest axial length in the fibrous layer represents the long axis, and the axis representing the shortest axial length represents the short axis, and both ends of the long axis and the short axis indicate vertices.

The thickness of the fibrous layer is largest at the horizontal long axis portion 111, as it approaches the vertex of the long axis, the thickness of the fibrous layer of horizontal short axis portion 112 decreases, and the thickness of the fibrous layer of horizontal short axis portion 112 is the smallest at the vertex of the long axis.

In the present specification, the thickness of the head part on which the fibrous layer is formed indicates the length of the vertical axis with respect to the longitudinal axis of the length direction of the swab in the head part on which the fibrous layer is formed.

According to an embodiment, the nostril swab and/or saliva swab includes a support part and an elliptical head part with a fibrous layer, the head part having a horizontal long axis portion and a horizontal short axis portion, wherein the thickness of the head part is 3.8-6.0 mm at the horizontal long axis portion and 2.0-3.5 mm at the horizontal short axis portion.

More specifically, the nostril swab and/or saliva swab includes a support part and an elliptical head part with a fibrous layer, the head part having a horizontal long axis portion and a horizontal short axis portion, and wherein the thickness of the head part is 4.8-5.2 mm at the horizontal long axis portion and 2.8-3.2 mm at the horizontal short axis portion.

According to an embodiment, fibers used in the formation of the fibrous layer may have a Dtex value of 3.00-3.50 and, more specifically, a Dtex value of 3.30-3.35.

The above-described head part thicknesses and Dtex values allow the nostril swab and/or saliva swab to have appropriate absorbing ability and, when dipped in a sample transport medium, to have appropriate releasing ability.

The fibers of the fibrous layer are a substance having hydrophilic properties, and examples thereof include polyesters, polyamides, and cotton. The fibrous layer may be formed by various methods known in the art, and for example, the fibrous layer may be formed by a flocking method.

The nostril swab and/or saliva swab completing sample collection is dipped in, that is, impregnated in a sample transport medium in the container. The sample contained in the swab is released to the sample transport medium by such dipping. The container containing the sample transport medium in which the swab has been dipped is usually transported to a site where a nucleic acid amplification reaction is performed.

The sample transport medium usable in the present invention includes two types of buffers: a cell maintenance buffer and a cell inactivation buffer (*i.e.*, a cell lysis buffer).

As used herein, the term "cell maintenance buffer" refers to a buffer that maintains cells in a sample not to be disrupted.

The cell maintenance buffer usable in the present invention includes any cell maintenance medium that is commonly used in the art. According to an embodiment, the cell maintenance buffer is a saline-based solution or a balanced salt solution-based buffer.

More specifically, the saline-based solution used in the present invention is phosphate buffered saline (PBS) or normal saline.

The balanced salt solution-based buffer used in the present invention may contain a balanced salt solution that is commonly used in the art. Examples of the balanced salt solutions include Alsever's solution, Earle's balanced salt solution, Gey's balanced salt solution, Hanks' balanced salt solution, Puck's balanced salt solution, Ringer's balanced salt solution, Simm's balanced salt solution, and Tyrode's balanced salt solution.

The balanced salt solution-based buffer used in the present invention may further contain other ingredients in addition to the above-descried balanced salt solutions. For example, examples of the other ingredients include bovine serum albumin, cysteine, sucrose, and glutamic acid.

The cell inactivation buffer, that is, cell lysis buffer, used in the present invention lyses a sample, for example, a virus to allow a nucleic acid molecule in the virus to be released into the buffer, denature protein components to inactivate DNase and RNase, and maintain the integrity of the nucleic acid molecule.

More specifically, the cell inactivation buffer as a sample transport medium used in the present invention contains (i) a chaotropic agent and, more specifically, contains, in addition to (i) the chaotropic agent, at least one ingredient selected from the group consisting of (ii) a detergent, (iii) a reducing agent, and (iv) a chelator, and still more specifically, contains, in addition to (i) the chaotropic agent, (ii) a detergent, (iii) a reducing agent, and (iv) a chelator. Alternatively, the cell inactivation buffer may further comprise (v) a buffer.

Examples of the chaotropic agent include guanidine thiocyanate, guanidine isocyanate, guanidine hydrochloride, and potassium thiocyanate, and more specifically, guanidine thiocyanate. The chaotropic agent opens microbial cells to induce cell lysis and allow the release of DNA and RNA, and inhibits the degradation of nucleic acid molecules by nucleases.

Specifically, the detergent is sodium dodecyl sulfate, lithium dodecyl sulfate, sodium taurodeoxycholate, sodium taurocholate, sodium glycocholate, sodium deoxycholate, sodium cholate, sodium alkylbenzene sulfonate, polysorbate, Triton X-100, or N-lauroyl sarcosine.

Specifically, the chelator is ethylene glycol tetraacetic acid, hydroxyethylethylenediaminetriacetic acid, diethylene triamine pentaacetic acid, N,N-bis(carboxymethyl)glycine, ethylenediaminetetraacetic, citrate anhydrous, sodium citrate, calcium citrate, ammonium citrate, ammonium bicitrate, citric acid, diammonium citrate, ferric ammonium citrate, or lithium citrate.

Specifically, examples of the buffer include tris(hydroxymethyl)aminomethane, citrates, 2-(N-morpholino)ethanesulfonic acid, N,N-Bis(2-hydroxyethyl)-2-aminoethanesulfonic acid, 1,3-bis(tris(hydroxymethyl)methyl amino)propane, 4-(2-hydroxyethyl)-1-piperazine ethanesulfonic acid, 3-(N-morpholino)propanesulfonic acid, hydroxyethyl piperazine ethane sulfonic acid, bicarbonates, and phosphates.

Specifically, examples of the reducing agent include 2-mercaptoethanol, tris(2-carboxyethyl)phosphine, dithiothreitol, dimethylsulfoxide, and sodium hydroxide.

In the sample transport medium used in the present invention, the chaotropic agent may be contained in an amount of 5-30 parts by weight relative to 100 parts by weight of the medium, and the detergent, the chelator, the buffer, and the reducing agent may be contained in amounts of 1-15 parts by weight, 0.1-5 parts by weight, 2-10 parts by weight, and 0.1-3 parts by weight, respectively.

According to an embodiment, the cell inactivation buffer as a sample transport medium has an inactivation function by lysis of a respiratory infection pathogen and a stabilization function of a nucleic acid material (specifically, DNA or RNA, and more specifically RNA) released from the lysed pathogen.

Optionally, the sample transport medium further contains a pH indicator (e.g., phenol red, bromocresol purple, and bromothymol blue), and more specifically phenol red. The pH indicator makes it possible to monitor whether the sample transport medium is infected or not.

A nucleic acid amplification reaction is performed using the sample transport medium. The sample transport medium may be applied to a nucleic acid amplification reaction through the following three methods:
According to the first method, the sample transport medium is directly applied to a nucleic acid amplification reaction without any pretreatment. For example, the sample transport medium may be added to a nucleic acid amplification reaction composition to perform PCR by a conventional direct PCR method.

According to the second method, the sample transport medium is subjected to heat treatment (incubation), and then the incubation resultant is applied to a nucleic acid amplification reaction.

Specifically, the sample transport medium having both the nostril swab sample and the saliva sample dipped therein is subjected to incubation at 60-100°C (more specifically, 95-100°C) for 1-25 minutes (more specifically, for 1-15 minutes) before use in the nucleic acid amplification reaction, and the nucleic acid amplification reaction is performed using the incubation resultant. In such a case, the sample transport medium used for the nucleic acid amplification reaction may be diluted 2 to 10 times (more specifically, 3 to 5 times) before or after the incubation, and then applied to the nucleic acid amplification reaction.

According to an embodiment, the sample transport medium having both the nostril swab sample and the saliva sample dipped therein is subjected to incubation at 60-100°C (more specifically, 95-100°C) for 1-25 minutes (more specifically, for 1-15 minutes). The samples may be lysed through the incubation.

According to an embodiment, the sample transport medium having both the nostril swab sample and the saliva sample dipped therein may be dispensed into a container and then incubated. For example, 5 µl to 40 µl of the sample transport medium having both the nostril swab sample and the saliva sample may be dispensed into the container.

According to another embodiment, the transport medium containing the samples may be incubated in a state in which the samples are contained in the original container where the samples have been collected and stored, without separate dispensing. An aliquot of the transport medium may be mixed with a nucleic acid amplification reaction composition.

According to an embodiment, the sample transport medium containing the samples may be used for incubation without dilution.

According to another embodiment, the sample transport medium containing the samples may be diluted with water or a buffer before incubation. For example, when incubated without separate dispensing, the sample transport medium containing the samples may be diluted by addition of water or a buffer; and when the sample transport medium containing the samples is dispensed into a container and incubated, water or a buffer for dilution may be contained in advance in the container, in which the samples are to be contained, or the sample transport medium containing the samples may be dispensed into the container, followed by addition with water or a buffer.

The dilution attenuates the inhibition action of a reaction inhibitor that may be present in the sample transport medium when the sample transport medium is mixed and reacted with the nucleic acid amplification reaction composition.

In an embodiment, a highly viscous sample (e.g., saliva) is diluted to have lowered viscosity and then incubated, thereby facilitating the lysis of the sample.

The water used for dilution may be, for example, purified water, nuclease-free-water (*e.g.*, RNase-free water), or distilled water.

The buffer used for dilution may be, for example, a TE buffer.

According to an embodiment, the sample transport medium used in the nucleic acid amplification reaction is diluted 2 to 10 times before application to the nucleic acid amplification reaction. The transport medium may be diluted, specifically, 2 to 8 times, 2 to 7 times, 2 to 6 times, 2 to 5 times, 2 to 4 times, or 3 to 5 times, and more specifically, 2 to 4 times or 3 to 5 times. For example, when the sample transport medium is diluted 2 times, the sample transport medium is mixed with water (or buffer) at 1:1 and thus diluted 2 times.

As used herein, the term "lysis of sample" includes the lysis of membranes or walls of cells, bacteria, viruses and the like contained in a sample *(e.g.,* a swab sample and saliva).

In an embodiment, the viscosity of the sample may mean the viscosity of a collected sample itself *(e.g.,* the viscosity of the nasal mucus itself put on a swab in a case of a swab sample), or the viscosity of a solution storing a collected sample *(e.g.,* a sample transport medium in which the nasal mucus put on a swab is dispersed).

According to an embodiment, the incubation temperature is 95°C or more, 96°C or more, 97°C or more, 98°C or more, or 99°C or more. According to an embodiment, the incubation temperature is 100°C or less or 99°C or less.

According to an embodiment, the incubation temperature is 97°C to 100°C, 97°C to 99°C, 97°C to 98°C, 98°C to 100°C, or 98°C to 99°C.

According to an embodiment, the incubation temperature is 98°C.

The incubation temperature may be selected to be a temperature at which a lysate of the sample can be obtained.

According to an embodiment, the incubation may be conducted for 1 minute or more, 2 minutes or more, or 3 minutes or more. According to an embodiment, the incubation may be conducted for 25 minute or less, 23 minutes or less, or 20 minutes or less.

According to an embodiment, the incubation may be conducted for 1 to 25 minutes, 1 to 20 minutes, 1 to 15 minutes, 2 to 25 minutes, 2 to 20 minutes, 2 to 15 minutes, 3 to 25 minutes, 3 to 20 minutes, or 3 to 15 minutes.

The incubation time may be selected as a time to prevent degradation of the target nucleic acid present in the sample at the incubation temperature.

For example, a relatively highly viscous sample is incubated for a longer time compared with a relatively low viscous sample, whereas a relatively low viscous sample is incubated for a shorter time compared with a relatively highly viscous sample, so that the degradation of a nucleic acid can be prevented.

According to an embodiment, when the sample is a swab sample, the incubation may be conducted for 2 to 4 minutes.

According to an embodiment, when the sample is a swab sample, the incubation may be conducted for 3 minutes.

According to an embodiment, when the sample is saliva, the incubation may be conducted for 18 to 22 minutes.

In an embodiment, when the sample is saliva, the incubation may be conducted for 20 minutes.

In an embodiment, a highly viscous sample *(e.g.,* saliva) is treated with proteinase K to have lowered viscosity and then incubated, thereby facilitating the lysis of the sample.

According to an embodiment, the membranes or walls of cells, bacteria, viruses and the like in samples can be lysed through solely the incubation at the selected temperature for reaction time, and thus the use of another substance for lysis is not required.

The incubation temperature and incubation time may be variously selected as long as the degradation of the target nucleic acid present in the sample is prevented while the membranes or walls of cells, bacteria, viruses and the like are lysed.

In an embodiment, the incubation may be conducted at 95°C to 100°C for 1 to 25 minutes.

In an embodiment, the incubation may be conducted at 95°C to 100°C for 2 to 20 minutes.

In an embodiment, the incubation may be conducted at 97°C to 100°C for 2 to 25 minutes.

In an embodiment, the incubation may be conducted at 97°C to 100°C for 2 to 20 minutes.

In an embodiment, the incubation may be conducted at 98°C to 100°C for 2 to 25 minutes.

In an embodiment, the incubation may be conducted at 98°C to 100°C for 2 to 20 minutes.

In an embodiment, the incubation may be conducted at 98°C for 2 to 25 minutes.

In an embodiment, the incubation may be conducted at 98°C for 2 to 20 minutes.

In an embodiment, when the sample is a swab sample, the incubation may be conducted at 97°C to 100°C for 2 to 4 minutes, specifically, may be conducted at 98°C for 3 minutes.

In an embodiment, when the sample is saliva, the incubation may be conducted at 97°C to 100°C for 18 to 22 minutes, and specifically, 98°C for 20 minutes.

The incubation temperature and incubation time selected herein correspond to conditions under which the lysate of a sample can be efficiently obtained without an additional nucleic acid extraction process and the degradation of a target nucleic acid can be inhibited. Furthermore, under such conditions, the target nucleic acid can be optimized in working as a template. Especially, when the target acid is RNA, the incubation temperature and incubation time selected herein correspond to conditions under which cDNA can be favorably prepared from target RNA.

In an embodiment, the incubation may be conducted with a volume of 10 µl to 60 µl. The volume may be a volume of the sample transport medium containing the samples that is not diluted, or a volume of the sample transport medium that is diluted.

In one embodiment, the method may be implemented by dispensing using an automated dispensing system (e.g., a liquid handler). For example, dispensing may be conducted using a liquid handler at a flow rate of 10 µl/s to 400 µl/s, and specifically, 10 µl/s to 300 µl/s, 10 µl/s to 200 µl/s, 10 µl/s to 100 µl/s, 20 µl/s to 100 µl/s, 20 µl/s to 80 µl/s, 20 µl/s to 70 µl/s, 30 µl/s to 80 µl/s, 30 µl/s to 70 µl/s, or 30 µl/s to 60 µl/s, but is not limited thereto.

According to the third method, the sample transport medium having both the nostril swab sample and the saliva sample dipped therein is subjected to a nucleic acid isolation process before use for a nucleic acid amplification reaction, and the nucleic acid amplification reaction is performed using a nucleic acid molecule isolated in the nucleic acid isolation process.

The nucleic acid isolation process may be conducted by, for example, a magnetic particle-based separation method (see U.S. Patent Nos. 6027945 and 7517969). For example, the nucleic acid molecule can be isolated by sequentially using buffers for nucleic acid molecule isolation, including a lysis buffer, a binding buffer, a washing buffer, and an eluting buffer. More specifically, the sample transport medium is treated with a lysis buffer containing a chaotropic agent (e.g., guanidine thiocyanate) and a detergent (e.g., Tween-20) to lyse viruses or bacteria in the sample, the lysis resultant is treated with a binding buffer containing magnetic particles to bind the nucleic acid molecule to the surface of the magnetic particles, and then the magnetic particles are washed with a washing buffer containing ethanol, and then treated with an eluting buffer, thereby separating the nucleic acid molecule bound to the surface of the magnetic particles.

In the nucleic acid amplification reaction, a probe and primers hybridizing with a target may be used.

The probe or primer used in the present invention has a sequence complementary to the target nucleotide sequence. As used herein, the term "complementary" refers to having complementarity to be selectively hybridizable with the above-described nucleotide sequence under specific hybridization or annealing conditions. Therefore, the term "complementary" has a different meaning from the term "perfectly complementary", and the primers or probe of the present invention may have one or more mismatch nucleotide sequences as long as the primers or probe is selectively hybridizable with the above nucleotide sequence.

As used herein, the term "primer" refers to a single-strand oligonucleotide capable of acting as an initial point of template-directed DNA synthesis at an appropriate temperature in an appropriate buffer under appropriate conditions (*i.e.*, four types of different nucleoside triphosphates and polymerase). The appropriate length of the primer may vary according to various factors, for example, temperature and a use of the primer, but the primer is typically 15 to 40 nucleotides in length. A short primer molecule generally requires a lower temperature in order to form a sufficiently stable hybrid complex together with a template.

The primer sequence is not required to be perfectly complementary to a partial sequence of the template, and it is enough that the primer sequence has sufficient complementarity within a range in which the primer can make an intrinsic action when hybridized with the template. Therefore, the primer of the present invention does not need to have a perfectly complementary sequence to the above-described nucleotide sequence, which is the template, and it is enough that the primer has a sufficient complementarity within a range in which the primer can perform a primer action when hybridized with the gene sequence. Such a primer can be easily designed with reference to the above-described nucleotide sequence by a person skilled in the art, and for example, the design can be carried out using a computer program for primer design (e.g., PRIMER 3 program).

As used herein, the term "probe" refers to a linear oligomer of natural or modified monomers or linkages, and the probe includes deoxyribonucleotides and ribonucleotides, can specifically hybridize with a target nucleotide sequence, and is naturally present or artificially synthesized. The probe of the present invention is preferably in a single-stranded form, and is an oligodeoxyribonucleotide.

As used herein, the term "amplification" refers to a reaction for amplifying nucleic acid molecules. A variety of amplification reactions have been reported in the art, including polymerase chain reaction (PCR, U.S. Patent Nos. 4,683,195, 4,683,202 and 4,800,159), reverse transcription-polymerase chain reaction (RT-PCR, Sambrook et. al., Molecular Cloning. A Laboratory Manual, 3rd ed. Cold Spring Harbor Press (2001)), methods by Miller, H. I. (WO 89/06700) and Davey, C. et al. (EP 329,822), ligase chain reaction (LCR), Gap-LCR (WO 90/01069), repair chain reaction (EP 439,182), transcription-mediated amplification (TMA, WO 88/10315), self-sustained sequence replication (WO 90/06995), selective amplification of target polynucleotide sequences (U.S. Patent No. 6,410,276), consensus sequence primed polymerase chain reaction (CP-PCR, U.S. Patent No. 4,437,975), arbitrarily primed polymerase chain reaction (AP-PCR, U.S. Patent Nos. 5,413,909 and 5,861,245), nucleic acid sequence based amplification (NASBA, U.S. Patent Nos. 5,130,238, 5,409,818, 5,554,517, and 6,063,603), strand displacement amplification, and loop-mediated isothermal amplification (LAMP), but is not limited thereto. Other usable amplification methods are disclosed in U.S. Patent Nos. 5,242,794, 5,494,810, and 4,988,617, and U.S. Patent Application Serial No. 09/854,317.

PCR is the most well-known method for nucleic acid amplification, and modifications and applications thereof have been developed. For example, for improving PCR specificity or sensitivity, touchdown PCR, hot start PCR, nested PCR, and booster PCR have been developed by modifying the conventional PCR procedure. In addition, real-time PCR, differential display PCR (DD-PCR), rapid amplification of cDNA ends (RACE), multiplex PCR, inverse polymerase chain reaction (IPCR), vectorette PCR, and thermal asymmetric interlaced PCR (TAIL-PCR) have been developed for specific applications. The details of PCR are disclosed in McPherson, M. J., and Moller, S.G. PCR. BIOS Scientific Publishers, Springer-Verlag New York Berlin Heidelberg. N.Y. (2000).

Most of respiratory pathogens have RNA as genome. In such a case, a gene amplification reaction is performed using primers to be hybridized with RNA or cDNA while RNA in a sample is used as a template. cDNA is synthesized from isolated RNA, and this cDNA is amplified. cDNA can be easily synthesized using reverse transcriptase (see PNAS USA, 85:8998(1988); Libert F, et al., Science, 244:569(1989); and Sambrook, J. et al., Molecular Cloning. A Laboratory Manual, 3rd ed. Cold Spring Harbor Press (2001)). Then, the synthesized cDNA is amplified through a gene amplification reaction.

The primers used for the present invention are hybridized or annealed to a region of the template to form a double-chain structure. Nucleic acid hybridization conditions suitable for forming such a double-chain structure are disclosed in Joseph Sambrook, et al., Molecular Cloning, A Laboratory Manual, Cold Spring Harbor Laboratory Press, Cold Spring Harbor, N. Y. (2001), and Haymes, B. D., et al., Nucleic Acid Hybridization, A Practical Approach, IRL Press, Washington, D.C. (1985).

A variety of DNA polymerases may be used in the amplification of the present invention, and include "Klenow" fragment of *E. coli* DNA polymerase I, a thermostable DNA polymerase, and bacteriophage T7 DNA polymerase. Preferably, the polymerase is a thermostable DNA polymerase obtained from a variety of bacterial species, including Thermus aquaticus (Taq), Thermus thermophilus (Tth), Thermus filiformis, Thermis flavus, Thermococcus literalis, and Pyrococcus furiosus (Pfu).

When a polymerization reaction is conducted, the ingredients necessary for the reaction are provided in excess to a reaction container. The excess with respect to the ingredients necessary for the amplification reaction means amounts of the ingredients such that the amplification reaction is not substantially restricted by the concentrations of the ingredients. A cofactor, such as Mg²⁺; and dATP, dCTP, dGTP, and dTTP are required to be provided to the reaction mixture to such an extent that a desired degree of amplification can be achieved. All the enzymes used in the amplification reaction may be in an active state under the same reaction conditions. Indeed, a buffer allows all enzymes to be under conditions near optimal reaction conditions. Therefore, the amplification of the present invention may be performed in a single reactant without changing conditions, such as the addition of reactants.

According to an embodiment of the present invention, the nucleic acid amplification reaction used in the present invention is a real-time nucleic acid amplification reaction.

The real-time nucleic acid amplification reaction may be performed using a non-specific fluorescence dye that non-specifically intercalates into a duplex, which is an amplicon of the target nucleic acid sequence.

In addition, the real-time nucleic acid amplification reaction may use a probe specifically hybridizing with the target nucleic acid sequence. Examples of the method include a molecular beacon method using a dual-labeled probe forming a hair-pin structure (Tyagi et al, Nature Biotechnology v. 14 Mar. 1996), a hybridization probe method using two probes each single-labeled with a donor or an acceptor (Bemad et al, 147-148 Clin Chem 2000; 46), a Lux method using a single-labeled oligonucleotide (U.S. Patent No. 7,537,886), and a TaqMan method using a cleavage reaction of a double-labeled probe by 5'-nuclease activity of DNA polymerase as well as the hybridization of a dual-labeled probe (U.S. Patent No. 5,210,015 and No. 5,538,848), but are not limited thereto.

In addition, the real-time nucleic acid amplification reaction may be conducted using a duplex dependently formed on the presence of the target nucleic acid sequence. The duplex dependently formed on the presence of the target nucleic acid sequence is not an amplicon itself of the target sequence formed by the amplification reaction, and the amount of the duplex increases in proportion to the amplification of the target nucleic acid sequence. The duplex formed depending on the presence of the target nucleic acid sequence may be obtained by various methods, for example, PTO cleavage and extension (PTOCE) disclosed in WO 2012/096523.

According to an embodiment of the present invention, the nucleic acid amplification reaction composition contains primers, a probe, and enzymes. The nucleic acid amplification reaction composition contains a buffer, dNTP, KCl, and MgCl₂. For example, the nucleic acid reaction composition may contain 40 mM Tris • Cl (pH 8.8), 100 mM KCl, 4 mM MgCl₂, and 400 µM dNTP. The enzymes contained in the nucleic acid amplification reaction composition of the present invention include *Taq* DNA polymerase (specifically, hot-start *Taq* DNA polymerase) and reverse transcriptase, and more specifically hot-start *Taq* DNA polymerase, reverse transcriptase, uracil DNA glycosylase (UDG), and RNase inhibitor (RI). The hot start *Taq* DNA polymerase includes chemically modified hot start *Taq* DNA polymerase (see: Paul N, et al., Hot start PCR. Methods in Molecular Biology. Humana Press. 630:301-18(2010)), antibody-based hot start *Taq* DNA polymerase (Paul N, et al., Hot start PCR. Methods in Molecular Biology. Humana Press. 630:301-18(2010)), and aptamer-based hot start *Taq* DNA polymerase (Birch DE, et al., Simplified hot start PCR. Nature 381:445-446(1996)). The reverse transcriptase used in the present invention is a reverse transcriptase that is conventionally used in the art, and specifically, MMLV-based thermally stable reverse transcriptase.

According to an embodiment of the present invention, the nucleic acid amplification reaction composition contains primers, a probe, and enzymes (hot start *Taq* DNA polymerase, MMLV-based thermally stable reverse transcriptase, and UDG).

An internal control (IC) is generally used in the nucleic acid amplification reaction to determine the validity of the nucleic acid amplification reaction. In the present invention, the sampling validity of nostril swab samples and saliva samples as well as the validity of nucleic acid amplification reactions can be determined by using two types of ICs.

According to an embodiment, the nucleic acid amplification reaction composition contains primers for amplifying the nucleic acid molecule of the respiratory pathogen, endogenous IC, and exogenous IC, and the endogenous IC is used to determine the sampling validity (validity) of the nostril swab sample and the saliva sample and the exogenous IC is used to determine the validity of the nucleic acid amplification reaction.

According to an embodiment of the present invention, the endogenous IC is RNase P gene, betaglobin gene, GAPDH gene, beta actin gene, PPIA gene, RPS9 gene, RPS15A gene, or UXT gene, and the exogenous IC is bacteriophage MS2 or armoured RNA.

### Step (b): Determination of presence or absence of respiratory pathogen

Finally, the presence or absence of the respiratory pathogen is determined by analyzing the results of the nucleic acid amplification reaction.

For example, when a signal above the particular threshold value is observed in the real-time detection of signals during the real-time nucleic acid amplification reaction, it is determined that a respiratory pathogen is present.

According to an embodiment of the present invention, the respiratory pathogen detected by the present invention is respiratory virus and/or respiratory bacteria.

For example, the present invention determines an infection by influenza virus (e.g., influenza A virus and influenza B virus), respiratory syncytial virus (RSV) (e.g., RSV A and RSV B), adenovirus, enterovirus, parainfluenza virus (PIV) *(e.g.,* PIV 1, PIV 2, PIV 3, and PIV 4), metapneumovirus (MPV), bocavirus, rhinovirus, coronavirus (e.g., CoV NL63, CoV 229E, CoV OC43, CoV HKU1, SARS-CoV, MERS-CoV, SARS-CoV-2, *Mycoplasma pneumoniae, Chlamydophila pneumoniae, Legionella pneumophila, Haemophilus influenzae, Streptococcus pneumoniae, Bordetella pertussis* and/or *Bordetella parapertussis.*

According to an embodiment of the present invention, the present invention determines an infection by respiratory virus including influenza virus, respiratory syncytial virus (RSV), adenovirus, enterovirus, parainfluenza virus (PIV), metapneumovirus (MPV), bocavirus, rhinovirus, and/or coronavirus. More specifically, the method of the present invention determines an infection by severe acute respiratory syndrome coronavirus 2 (SARS-CoV-2).

According to an embodiment, the nucleic acid amplification reaction composition contains primers, which are used to amplify at least one, more specifically at least two, and still more specifically three genes among RdRP gene, S gene, and N gene of SARS-CoV-2, and when at least one (more specifically at least two genes, still more specifically three genes) of the RdRP gene, S gene, and N gene is measured to be positive, it is determined that a pathogen of SARS-CoV-2 is present.

Alternatively, the nucleic acid amplification reaction composition contains primers, which are used to amplify at least one, more specifically at least two, still more specifically three and still much more specifically four genes among E gene, RdRP gene, S gene and N gene of SARS-CoV-2, and when at least one (more specifically at least two genes) of the E gene and N gene and at least one of RdRP gene and S gene are measured to be positive, it is determined that a pathogen of SARS-CoV-2 is present.

According to an embodiment of the present invention, the present invention is implemented by a real-time nucleic acid amplification reaction showing a limit of detection (LoD) value of 100-500 RNA copies/reaction.

In another aspect of the present invention, there is provided the use of a sampling kit for detecting a respiratory pathogen, the kit including: (a) a nostril swab; (b) a saliva sampling device; and (c) a container containing a sample transport medium.

Since the sampling kit is used for implementing the above-described method of the present invention, the common descriptions between them are omitted in order to avoid undue redundancy leading to the complexity of the present specification.

According to an embodiment of the present invention, the saliva sampling device is a saliva swab.

According to an embodiment, the sampling kit is a self-sampling kit.

According to an embodiment of the preset invention, the saliva swab is applied to a supralingual part or a sublingual part.

According to an embodiment, the nostril swab and/or saliva swab includes a support part and an elliptical head part with a fibrous layer, the head part having a horizontal long axis portion and a horizontal short axis portion, wherein the thickness of the head part is 3.8-6.0 mm at the horizontal long axis portion and 2.0-3.5 mm at the horizontal short axis portion.

According to an embodiment, the nostril swab and/or saliva swab includes a support part and an elliptical head part with a fibrous layer, the head part having a horizontal long axis portion and a horizontal short axis portion, and wherein the thickness of the head part is 4.8-5.2 mm at the horizontal long axis portion and 2.8-3.2 mm at the horizontal short axis portion.

According to an embodiment of the present invention, the fibers of the fibrous layer have a Dtex value of 3.00-3.50. More specifically, the fibers of the fibrous layer have a Dtex value of 3.30-3.35.

According to an embodiment, the sample transport medium is a cell maintenance buffer or a cell inactivation buffer.

According to an embodiment of the present invention, the cell maintenance buffer is a saline-based solution or a balanced salt solution-based buffer.

According to an embodiment, the saline-based solution is phosphate buffered saline (PBS) or normal saline.

According to an embodiment, the cell inactivation buffer contains (i) a chaotropic agent. More specifically, the cell inactivation buffer further contains at least one ingredient selected from the group consisting of (ii) a detergent; (iii) a reducing agent; and (iv) a chelator. Alternatively, the cell inactivation buffer may further comprise (v) a buffer.

According to an embodiment of the present invention, when a sample sampled by the nostril swab and the saliva sampling device is impregnated in the sample transport medium, the sample transport medium is used in the nucleic acid amplification reaction without a nucleic acid isolation process.

Optionally, the sample transport medium further contains a pH indicator (e.g., phenol red, bromocresol purple, and bromothymol blue).

In still another aspect of the present invention, there is provided the use of a kit for detecting a respiratory pathogen, the kit including:
(a) the sampling kit described above; and
(b) a real-time nucleic acid amplification reaction composition containing: (i) primers for amplifying a nucleic acid molecule of the respiratory pathogen; (ii) a probe hybridizing with the nucleic acid molecule of the respiratory pathogen; and (iii) enzymes including DNA polymerase.

Since the kit for detecting a respiratory pathogen is used for implementing the above-described method of the present invention, the common descriptions between them are omitted in order to avoid undue redundancy leading to the complexity of the present specification.

According to an embodiment of the present invention, the enzyme includes hot start *Taq* polymerase and reverse transcriptase.

According to an embodiment, the nucleic acid amplification reaction composition further contains primers for amplifying endogenous IC and exogenous IC. According to an embodiment of the present invention, the endogenous IC is RNase P gene, betaglobin gene, GAPDH gene, beta actin gene, PPIA gene, RPS9 gene, RPS15A gene, or UXT gene, and the exogenous IC is bacteriophage MS2 or armoured RNA.

According to an embodiment, the respiratory pathogen includes respiratory viruses and/or respiratory bacteria. More specifically, the respiratory virus includes influenza viruses, respiratory syncytial viruses (RSV), adenoviruses, enteroviruses, parainfluenza viruses (PIV), metapneumoviruses (MPV), bocaviruses, rhinoviruses and/or coronaviruses. Still more specifically, the coronavirus is severe acute respiratory syndrome coronavirus 2 (SARS-CoV-2).

According to an embodiment of the present invention, the nucleic acid molecule of the respiratory pathogen is at least one gene of RdRP gene, S gene, or N gene of SARS-CoV-2.

In another aspect of the present invention, there is provided a sampling method for detecting a respiratory pathogen, the method including:
(a) obtaining a nostril swab sample and a saliva sample by using the sampling kit of the present invention; and
(b) impregnating the nostril swab sample and the saliva sample with a sample transport medium contained in one container.

Since the sampling method of the present invention uses the above-described sampling kit for implementing the method, the common descriptions between them are omitted in order to avoid undue redundancy leading to the complexity of the present specification.

The features and advantages of the present invention are summarized as follows.
(a) Nasopharyngeal swabbing, which is a conventional method, has problems that a separate swab device is required, sampling can be achieved by only an expert, and a sampler is exposed to a high risk of infection due to the adoption of aerosol-generating sampling manner. However, the swab sample and saliva sample (*e.g.,* a saliva swab sample) in the present invention can be obtained through self-sampling by an ordinary person and through a non-aerosol-generating sampling manner, and thus the safety and convenience of sampling can be significantly improved.
(b) The use of both the nostril swab sample and the saliva sample (e.g., saliva swab sample) shows a positive rate equivalent to that in nasopharyngeal swab sample, which is a conventional method, and allows self-sampling.
(c) The method of the present invention may be implemented without nucleic acid isolation, thereby relieving problems resulting from nucleic acid isolation (a prolonged testing time and a need for reagents and equipment for nucleic acid isolation).

### EXAMPLES

SARS-CoV-2 detection was conducted according to the protocol presented in the present invention.

Specifically, Real-time reverse-transcription PCR test (using Allplex^{™} SARS-CoV-2 Assay and Allplex^{™} SARS-CoV-2/FluA /FluB/RSV Assay from Seegene, Inc.) was performed on isolated nucleic acids or crude nucleic acids by collecting nasopharyngeal swab samples and Combo-swab (a combination of nostril swab samples and saliva swab samples) from patients suspected of SARS-CoV-2 infection or patients who were positive after SARS-CoV-2 test results at Gyeongsang National University Changwon Hospital in Korea (70 positives and 30 negatives). The clinical performance of each type of samples was compared in detecting SARS-CoV-2, and the test results of the Combo swab sample self-collected by the patients and the test results of the Combo swab sample collected by medical staffs (experts) were compared.

The nasopharyngeal swab sample was collected from patients by the method used in hospitals.

A nostril swab sample and a saliva swab sample were collected as follows: The nostril swab and saliva swab devices enclosed in ALLTM (SG Medical Inc.) were used to collect samples.

A nostril swab sample was obtained by inserting the nostril swab by 2-3 cm into the nostrils and swabbing the nostrils while smoothly rotating the nostril swab for 20 seconds. One nostril was swabbed and then the other nostril was swabbed. Then, a saliva swab sample was obtained by applying the saliva swab to the sublingual part and swabbing the sublingual part to allow saliva to be sufficiently absorbed into the fibrous layer of the swab. When the saliva swab sample was not obtained in a sufficient amount, it was additionally obtained from the inside of cheek, the gingiva and/or palate by using the saliva swab. Then, both the nostril swab sample and the saliva swab sample which were collected by experts were impregnated in a sample transport medium (ALLTM, SG Medical Inc.) as a Hank's balanced salt-based medium to preserve viral integrity. Both the nostril swab sample and the saliva swab sample which were collected by patients were impregnated in saline medium.

The nasopharyngeal swab sample was also impregnated in a sample transport medium (ALLTM, SG Medical Inc.).

Then, the sample transport medium was subjected to heat treatment or nucleic acid isolation to obtain a target nucleic acid to be added to RT-PCR reactants.

To obtain the target nucleic acid by heat treatment (hereinafter referred to as "crude nucleic acid"), the sample transport medium impregnated with each of the nasopharyngeal swab samples and combo swab samples was diluted three times, and incubated at 98°C for 3 minutes, thereby obtaining the target nucleic acid.

Nucleic acid isolation was conducted using Seegene's STARMag 96X4 Universal Cartridge Kit (Cat. No. 744300.4. UC384, Seegene Inc.) and the automated nucleic acid extraction system Microlab NIMBUS (Cat. No. 65415-02, Hamilton). The nucleic acid isolation was conducted according to the manuals from the manufacturers of nucleic acid isolation reagents and the operation manual of the system. The nucleic acid isolation was conducted using 300 µl of the sample transport medium, in which each of the nasopharyngeal swab samples and combo swab samples was impregnated (hereinafter, referred to as "isolated nucleic acid").

The RT-PCR reaction using the crude nucleic acid or isolated nucleic acid was performed by Allplex^{™} SARS-CoV-2 Assay (Seegene Inc.) or Allplex^{™} SARS-CoV-2/FluA/FluB/RSV Assay (Seegene Inc.), which is a multiplex one-step RT-PCR product. The Allplex^{™} SARS-CoV-2 Assay product is a one-step RT-PCR product for detecting a target gene (E gene) of both Sarbecovirus and SARS-CoV-2 and target genes (RdRP gene, S gene, and N gene) of SARS-CoV-2. The Allplex^{™} SARS-CoV-2/FluA/FluB/RSV Assay product is a one-step RT-PCR product for detecting target genes of SARS-CoV-2 (RdRP gene, S gene, and N gene), influenza A virus, influenza B virus and respiratory syncytial virus.

When using Allplex^{™} SARS-CoV-2 Assay product, a reaction mixture for the RT-PCR reaction having a final volume of 20 µl was prepared using 5 µl of the crude nucleic acid or the isolated nucleic acid, 5 µl of an enzyme (containing chemically modified hot start Taq polymerase, RTase, UDG, and RI), 5 µl of RNase-free Water, and 5 µl of SARS2 MOM (Oligo mix). Tubes each containing the reaction mixture were placed in a real-time thermocycler (CFX96, Bio-Rad), and then reacted at 50°C for 20 minutes, and preactivated at 95°C for 15 minutes, followed by repeating 45 cycles of 10 seconds at 95°C, 15 seconds at 60°C, and 10 seconds at 72°C. The test was repeated twice to obtain an average Ct value.

In Allplex SARS-CoV-2 Assay, its analytes and assay result interpretations are summarized as follows:

**Table 1a**

| **Fluorophores** | **Analytes** |
|---|---|
| FAM | E gene |
| HEX | Internal Control (IC) |
| Cal Red 610 | RdRP/S gene |
| Quasar 670 | N gene |

**Table 1b**

| **Analytes** | **Ct value** | **Result** |
|---|---|---|
| Targets | ≤ 40 | Detected (+) |
| | > 40 | Not detected (-) |
| Internal Control (IC) | ≤ 40 | Detected (+) |
| | > 40 | Not detected (-) |

**Table 1c**

| **Target** | **Case 1** | **Case 2** | **Case 3** | **Case 4** | **Case 5** | **Case 6** | **Case 7** |
|---|---|---|---|---|---|---|---|
| **IC** | +/- | +/- | +/- | +/- | +/- | + | - |
| **E gene** | + | +/- | - | +/- | + | - | - |
| **RdRP/S gene** | + | - | + | + | - | - | - |
| **N gene** | + | + | + | - | - | - | - |
| **Interpretation** | SARS-CoV-2 Detected | SARS-CoV-2 Inconclusive | | | | SARS-CoV-2 Not detected | Invalid |

When using Allplex^{™} SARS-CoV-2/FluA/FluB/RSV Assay product, a reaction mixture for the RT-PCR reaction having a final volume of 20 µl was prepared using 5 µl of the crude nucleic acid, 5 µl of an enzyme (containing chemically modified hot start Taq polymerase, RTase, UDG, and RI), 5 µl of RNase-free Water, and 5 µl of SC2FabR MOM (Oligo mix), and a reaction mixture for the RT-PCR reaction having a final volume of 20 µl was prepared using 10 µl of the isolated nucleic acid, 5 µl of an enzyme (containing chemically modified hot start Taq polymerase, RTase, UDG, and RI), and 5 µl of SC2FabR MOM (Oligo mix). Tubes each containing the reaction mixture were placed in a real-time thermocycler (CFX96, Bio-Rad), and then reacted at 50°C for 20 minutes, preactivated at 95°C for 15 minutes, and repeated 3 cycles of 10 seconds at 95°C, 40 seconds at 60°C, and 20 seconds at 72°C, followed by repeating 42 cycles of 10 seconds at 95°C, 15 seconds at 60°C, and 10 seconds at 72°C. The signal detections were performed both at 60°C and 72°C at each cycle. The test was repeated twice to obtain an average Ct value.

In Allplex SARS-CoV-2/FluA/FluB/RSV Assay, its analytes and assay result interpretations are summarized as follows:

**Table 1d**

| **Fluorophores** | **Analytes** | |
|---|---|---|
| | **Graph 1** | **Graph 2** |
| FAM | S gene | RSV |
| HEX | RdRP gene | Flu B |
| Cal Red 610 | N gene | Flu A |
| Quasar 670 | Endo IC | Exo IC |

The Allplex SARS-CoV-2/FluA/FluB/RSV Assay product has been developed by using PTOCE technology (WO 2012/096523). Graph 1 means a real-time PCR amplification curve detected at 60°C and Graph 2 means a real-time PCR amplification curve detected at 72°C.

**Table 1e**

| **Results** | **Targets** | | | | | | | |
|---|---|---|---|---|---|---|---|---|
| | **FAM (Cₜ)** | | **HEX (Cₜ)** | | **Cal Red 610 (Cₜ)** | | **Quasar 670 (Cₜ)** | |
| | **S gene** | **RSV** | **RdRP** | **Flu B** | **N gene** | **Flu A** | **Endo IC** | **Exo IC** |
| Detected (+) | ≤ 38 | ≤ 38 | ≤ 40 | ≤ 38 | ≤ 38 | ≤ 38 | ≤ 38 | ≤ 38 |
| Not Detected (-) | > 38 | > 38 | > 40 | > 38 | > 38 | > 38 | > 38 | > 38 |

**Table 1f-1**

| **S gene** | **RdRP gene** | **N gene** | **SARS-CoV-2 Result Interpretation** |
|---|---|---|---|
| + | + | + | SARS-CoV-2 Detected |
| + | + | - | SARS-CoV-2 Inconclusive |
| + | - | + | |
| - | + | + | |
| + | - | - | |
| - | + | - | |
| - | - | + | |
| - | - | - | SARS-CoV-2 Not detected |

**Table 1f-2**

| **Target Result** | **Endo IC Result** | **Exo IC Result** | **Result Interpretation** |
|---|---|---|---|
| + | + | + | Target Detected |
| | - | + | |
| | + | - | |
| - | + | + | Target Not detected |
| | - | + | Invalid |
| | + | - | Invalid |
| +/- | - | - | Invalid |

The comparative results of clinical performance of combo swab samples compared to nasopharyngeal swab samples in SARS-CoV-2 detection are summarized in Table 2 below:

**Table 2**

| **Type of Samples** | **Assay** | **Type of nucleic acids** | **Clinical performance compared to nasopharyngeal swab samples** | | |
|---|---|---|---|---|---|
| | | | **sensitivity** | **specificity** | **overall agreement** |
| Combo swab | Assay 1 | Isolated | 91.38% | 100% | 94.32% |
| | | Crude | 82.93% | 100% | 90.14% |
| | Assay 2 | Isolated | 87.72% | 100% | 91.95% |
| | | Crude | 80.43% | 100% | 88.16% |
| Combo swab-self | Assay 1 | Isolated | 89.66% | 100% | 93.18% |
| | | Crude | 90.24% | 100% | 90.24% |
| | Assay 2 | Isolated | 84.21% | 100% | 89.66% |
| | | Crude | 73.91% | 100% | 84.21% |

In Table 2, the combo swab indicates the combo swab sample collected by medical staffs, and the combo swab-self indicates the combo swab sample self-collected by patients. And, the Allplex^{™} SARS-CoV-2 Assay was represented by Assay 1, and the Allplex^{™} SARS-CoV-2/FluA/FluB/RSV Assay was represented by Assay 2.

As a test result, the use of both the nostril swab sample and the saliva swab sample (combo swab sample) showed an equivalent positive rate for SARS-CoV-2 since for isolated nucleic acids, Assay 1 showed a 93.18-94,32% overall agreement, and Assay 2 showed a 89.66-91.95% overall agreement compared with the use of the nasopharyngeal swab sample.

The use of the isolated nucleic acid obtained from the nostril swab sample and the saliva swab sample (combo swab sample) can obtain a positive signal for SARS-CoV-2. In addition, the use of the crude nucleic acid from the nostril swab sample and the saliva swab sample (combo swab sample) can also obtain a positive signal for SARS-CoV-2, but indicated a slight decrease in sensitivity and overall agreement compared to the isolated nucleic acid. Such slightly reduced sensitivity and overall agreement would be interesting results considering that the crude nucleic acid rather than the isolated nucleic acid was used.

In the case of nasopharyngeal swab samples, there was discomfort in that the subject needs to visit a medical institution and the medical staffs must proceed with sample collection, since self-sampling is not practical. However, as can be seen from the results for combo swab-self in Table 1, the availability of self-collected combo swab samples was confirmed because there is a slight difference in sensitivity and overall agreement, but there is no problem in sample stability. In addition to this application, it is expected that the effect of reducing the movement of the subject, shortening the collection time, and reducing medical personnel will be achieved.

## Claims

1. A method for detecting a respiratory pathogen, the method comprising:
(a) performing a nucleic acid amplification reaction by using a sample transport medium in which a nostril swab sample and a saliva sample are dipped together and a nucleic acid amplification reaction composition; and
(b) analyzing the results of the nucleic acid amplification reaction to determine the presence or absence of the respiratory pathogen.

2. The method according to claim 1, wherein the saliva sample is a saliva swab sample.

3. The method according to claim **1,** wherein the nostril swab sample and the saliva sample are obtained through self-sampling; or wherein the nostril swab sample is obtained by applying one swab device to both of two nostrils.

4. The method according to claim 2, wherein the saliva swab sample is obtained by applying a swab device to a supralingual part or a sublingual part, preferably wherein the saliva swab sample is obtained by applying a swab device to the sublingual part.

5. The method according to claim 2, wherein the nostril swab sample and the saliva swab sample are obtained by applying two swab devices to the nostrils and a tongue, respectively; or wherein the nostril swab sample and the saliva swab sample are obtained by applying one swab device to the nostrils followed by a tongue.

6. The method according to claim 1, wherein the sample transport medium is a cell maintenance buffer or a cell inactivation buffer, preferably wherein the cell maintenance buffer is a saline-based solution or a balanced salt solution-based buffer.

7. The method according to claim 1, wherein the sample transport medium in which a nostril swab sample and a saliva sample are dipped together is subjected to a nucleic acid isolation process before use in the nucleic acid amplification reaction, and the nucleic acid amplification reaction is performed using a nucleic acid molecule isolated in the nucleic acid isolation process.

8. The method according to claim 1, wherein the sample transport medium in which a nostril swab sample and a saliva sample are dipped together is subjected to incubation at 60°C to 100°C for 1 to 25 minutes before use in the nucleic acid amplification reaction, and the nucleic acid amplification reaction is performed using the incubation resultant, preferably wherein the sample transport medium used in the nucleic acid amplification reaction is diluted 2 to 10 times before application to the nucleic acid amplification reaction.

9. The method according to claim 1, wherein the respiratory pathogen is respiratory virus and/or respiratory bacteria, preferably wherein the respiratory virus is influenza virus, respiratory syncytial virus (RSV), adenovirus, enterovirus, parainfluenza virus (PIV), metapneumovirus (MPV), bocavirus, rhinovirus and/or coronavirus.

10. The method according to claim 9, wherein the coronavirus is severe acute respiratory syndrome coronavirus 2 (SARS-CoV-2), preferably wherein the nucleic acid amplification reaction composition contains primers used for amplifying at least one of RdRP gene, S gene, and N gene of SARS-CoV-2.

11. The method according to claim 1, wherein the nucleic acid amplification reaction is a real-time nucleic acid amplification reaction; or wherein the nucleic acid amplification reaction composition contains primers, a probe, and an enzyme.

12. Use of a sampling kit for detecting a respiratory pathogen, the sampling kit comprising: (a) a nostril swab; (b) a saliva sampling device; and (c) a container containing a sample transport medium, preferably wherein the saliva sampling device is a saliva swab; wherein the sampling kit is a self-sampling kit; wherein the sample transport medium is a cell maintenance buffer or a cell inactivation buffer; or wherein when a sample sampled by the nostril swab and the saliva sampling device is impregnated in the sample transport medium, the sample transport medium is used in the nucleic acid amplification reaction without a nucleic acid isolation process, more preferably wherein the saliva swab is applied to a supralingual part or a sublingual part; or wherein the cell maintenance buffer is a saline-based solution or a balanced salt solution-based buffer.

13. Use of a kit for detecting a respiratory pathogen, the kit comprising:
(a) the sampling kit of claim 12; and
(b) a real-time nucleic acid amplification reaction composition containing (i) primers for amplifying a nucleic acid molecule of the respiratory pathogen; (ii) a probe hybridizing with the nucleic acid molecule of the respiratory pathogen; and (iii) an enzyme including DNA polymerase, preferably wherein the enzyme includes hot start *Taq* polymerase and reverse transcriptase; or wherein the respiratory pathogen is respiratory virus and/or respiratory bacteria, more preferably wherein the respiratory virus is influenza virus, respiratory syncytial virus (RSV), adenovirus, enterovirus, parainfluenza virus (PIV), metapneumovirus (MPV), bocavirus, rhinovirus and/or coronavirus.

14. Use of the kit according to claim 13, wherein the coronavirus is severe acute respiratory syndrome coronavirus 2 (SARS-CoV-2), preferably wherein the nucleic acid molecule of the respiratory pathogen is at least one gene of RdRP gene, S gene and N gene of SARS-CoV-2.

15. A sampling method for detecting a respiratory pathogen, the method comprising:
(a) obtaining a nostril swab sample and a saliva sample by using the sampling kit of claim 12; and
(b) impregnating the nostril swab sample and the saliva sample in a sample transport medium contained in one container.

## Patentansprüche

1. Verfahren zum Nachweis eines respiratorischen Krankheitserregers, wobei das Verfahren Folgendes umfasst:
(a) Durchführen einer Nukleinsäure-Amplifikationsreaktion unter Verwendung eines Probentransportmediums, in das ein Nasenabstrich und eine Speichelprobe zusammen eingetaucht werden, und einer Nukleinsäure-Amplifikationsreaktionszusammensetzung; und
(b) Analysieren des Ergebnisses der Nukleinsäure-Amplifikationsreaktion zur Bestimmung des Vorhandenseins oder Nichtvorhandenseins des respiratorischen Krankheitserregers.

2. Verfahren nach Anspruch 1, wobei die Speichelprobe ein Speichelabstrich ist.

3. Verfahren nach Anspruch 1, wobei der Nasenabstrich und die Speichelprobe durch einen Selbsttest erhalten werden; oder wobei der Nasenabstrich durch Anwendung einer Abstrichvorrichtung in den beiden Nasenlöchern erhalten wird.

4. Verfahren nach Anspruch 2, wobei die Speichelprobe durch Anwendung einer Abstrichvorrichtung in einem supralingualen oder sublingualen Bereich erhalten wird; wobei die Speichelprobe bevorzugt durch Anwendung einer Abstrichvorrichtung in dem sublingualen Bereich erhalten wird.

5. Verfahren nach Anspruch 2, wobei der Nasenabstrich und die Speichelprobe durch Anwendung von zwei Abstrichvorrichtungen jeweils in den Nasenlöchern und an einer Zunge erhalten werden; oder wobei der Nasenabstrich und die Speichelprobe durch Anwendung einer Abstrichvorrichtung in den Nasenlöchern und anschließend an einer Zunge erhalten werden.

6. Verfahren nach Anspruch 1, wobei das Probentransportmedium ein Zellerhaltungspuffer oder ein Zellinaktivierungspuffer ist, wobei der Zellerhaltungspuffer bevorzugt eine kochsalzbasierte Lösung oder ein ausgewogener salzlösungsbasierter Puffer ist.

7. Verfahren nach Anspruch 1, wobei das Probentransportmedium, in das ein Nasenabstrich und eine Speichelprobe zusammen eingetaucht werden, vor der Verwendung in der Nukleinsäure-Amplifikationsreaktion einem Nukleinsäure-Isolationsprozess unterzogen wird, und die Nukleinsäure-Amplifikationsreaktion unter Verwendung eines in dem Nukleinsäure-Isolationsprozess isolierten Nukleinsäuremoleküls durchgeführt wird.

8. Verfahren nach Anspruch 1, wobei das Probentransportmedium, in das ein Nasenabstrich und eine Speichelprobe zusammen eingetaucht werden, vor der Verwendung in der Nukleinsäure-Amplifikationsreaktion einer Inkubation bei 60° C bis 100° C für 1 bis 25 Minuten unterzogen wird, und die Nukleinsäure-Amplifikationsreaktion unter Verwendung des Inkubationsergebnisses durchgeführt wird, wobei das in der Nukleinsäure-Amplifikationsreaktion verwendete Probentransportmedium bevorzugt vor Zugabe zur Nukleinsäure-Amplifikationsreaktion 2 bis 10 Mal verdünnt wird.

9. Verfahren nach Anspruch 1, wobei der respiratorische Krankheitserreger ein respiratorisches Virus und/oder ein respiratorisches Bakterium ist, wobei das respiratorische Virus bevorzugt Influenzavirus, Respiratorisches Synzytial-Virus (RSV), Adenovirus, Enterovirus, Parainfluenzavirus (PIV), Metapneumovirus (MPV), Bocavirus, Rhinovirus und/oder Coronavirus ist.

10. Verfahren nach Anspruch 9, wobei das Coronavirus schweres akutes Atemwegssyndrom Coronavirus 2 (SARS-CoV-2) ist, wobei die Nukleinsäure-Amplifikationsreaktionszusammensetzung bevorzugt Primer enthält, die zur Amplifikation von mindestens einem von RdRP-Gen, S-Gen und N-Gen von SARS-CoV-2 verwendet werden.

11. Verfahren nach Anspruch 1, wobei die Nukleinsäure-Amplifikationsreaktion eine Echtzeit-Nukleinsäure-Amplifikationsreaktion ist; oder wobei die Nukleinsäure-Amplifikationsreaktionszusammensetzung Primer, eine Sonde und ein Enzym enthält.

12. Verwendung eines Test-Kits zum Nachweis eines respiratorischen Krankheitserregers, wobei der Test-Kit Folgendes umfasst:
(a) eine Vorrichtung zum Nasenabstrich; (b) ein Gerät zur Entnahme einer Speichelprobe; und (c) einen ein Probentransportmedium enthaltenen Behälter, wobei das Gerät zur Entnahme einer Speichelprobe bevorzugt eine Vorrichtung zum Speichelabstrich ist; wobei der Test-Kit ein Selbsttest-Kit ist; wobei das Probentransportmedium ein Zellerhaltungspuffer oder ein Zellinaktivierungspuffer ist; oder wobei, wenn eine durch den Nasenabstrich erhaltene Probe und das Gerät zur Entnahme der Speichelprobe im Probentransportmedium getränkt werden, das Probentransportmedium in der Nukleinsäure-Amplifikationsreaktion ohne einen Nukleinsäure-Isolationsprozess verwendet wird, wobei dir Vorrichtung zum Speichelabstrich weiter bevorzugt in einem supralingualen oder sublingualen Bereich angewendet wird; oder wobei der Zellerhaltungspuffer eine kochsalzbasierte Lösung oder ein ausgewogener salzlösungsbasierter Puffer ist.

13. Verwendung eines Kits zum Nachweis eines respiratorischen Krankheitserregers, wobei der Kit Folgendes umfasst:
(a) den Test-Kit nach Anspruch 12; und
(b) eine Echtzeit-Nukleinsäure-Amplifikationsreaktionszusammensetzung enthaltend (i) Primer zur Amplifikation eines Nukleinsäuremoleküls des respiratorischen Krankheitserregers; (ii) eine mit dem Nukleinsäuremolekül des respiratorischen Krankheitserregers hybridisierende Sonde; und (iii) ein Enzym mit DNA-Polymerase, wobei das Enzym bevorzugt Hot-Start-Taq-Polymerase und reverse Transkriptase umfasst; oder wobei der respiratorische Krankheitserreger ein respiratorisches Virus und/oder ein respiratorisches Bakterium ist, wobei das respiratorische Virus weiter bevorzugt Influenzavirus, Respiratorisches Synzytial-Virus (RSV), Adenovirus, Enterovirus, Parainfluenzavirus (PIV), Metapneumovirus (MPV), Bocavirus, Rhinovirus und/oder Coronavirus ist.

14. Verwendung des Kits nach Anspruch 13, wobei das Coronavirus schweres akutes Atemwegssyndrom Coronavirus 2 (SARS-CoV-2) ist, wobei das Nukleinsäuremolekül des respiratorischen Krankheitserregers bevorzugt mindestens ein Gen von RdRP-Gen, S-Gen und N-Gen von SARS-CoV-2 ist.

15. Testverfahren zum Nachweis eines respiratorischen Krankheitserregers, wobei das Verfahren umfasst:
(a) Erhalten eines Nasenabstrichs und einer Speichelprobe unter Verwendung des Test-Kits nach Anspruch 12; und
(b) tränken des Nasenabstrichs und der Speichelprobe in einem Probentransportmedium, das in einem Behälter enthalten ist.

## Revendications

1. Procédé de détection d'un agent pathogène respiratoire, ledit procédé comprenant :
(a) la réalisation d'une réaction d'amplification d'acide nucléique à l'aide d'un milieu de transport d'échantillon dans lequel un prélèvement nasal et un échantillon de salive sont trempés ensemble, ainsi que d'une composition pour réaction d'amplification d'acide nucléique ; et
(b) l'analyse des résultats de la réaction d'amplification d'acide nucléique afin de déterminer la présence ou l'absence de l'agent pathogène respiratoire.

2. Procédé selon la revendication 1, l'échantillon de salive étant un échantillon prélevé à l'aide d'un écouvillon.

3. Procédé selon la revendication 1, l'échantillon prélevé par écouvillonnage nasal et l'échantillon de salive étant obtenus par auto-prélèvement ; ou l'échantillon prélevé par écouvillonnage nasal étant obtenu en appliquant un seul dispositif d'écouvillonnage dans les deux narines.

4. Procédé selon la revendication 2, l'échantillon de salive prélevé à l'aide d'un écouvillon étant obtenu en appliquant un dispositif d'écouvillonnage sur une partie supralinguale ou une partie sublinguale, l'échantillon de salive prélevé à l'aide d'un écouvillon étant de préférence obtenu en appliquant un dispositif d'écouvillonnage sur la partie sublinguale.

5. Procédé selon la revendication 2, l'échantillon prélevé par écouvillonnage nasal et l'échantillon prélevé par écouvillonnage salivaire étant obtenus en appliquant respectivement d dispositifs d'écouvillonnage dans les narines et la langue ; ou l'échantillon prélevé par écouvillonnage nasal et l'échantillon prélevé par écouvillonnage salivaire étant obtenus en appliquant un dispositif d'écouvillonnage sur les narines, puis sur la langue.

6. Procédé selon la revendication 1, le milieu de transport de l'échantillon est un tampon de maintien cellulaire ou un tampon d'inactivation cellulaire, le tampon de maintien cellulaire étant de préférence une solution saline ou un tampon à base de solution saline équilibrée.

7. Procédé selon la revendication 1, le support de transport d'échantillons dans lequel un prélèvement nasal et un échantillon de salive sont placés ensemble étant soumis à un processus d'isolation d'acide nucléique avant d'être utilisé dans la réaction d'amplification d'acide nucléique, et la réaction d'amplification d'acide nucléique étant réalisée à l'aide d'une molécule d'acide nucléique isolée dans ledit processus d'isolation d'acide nucléique.

8. Procédé selon la revendication 1, le milieu de transport d'échantillons dans lequel un échantillon prélevé par écouvillonnage nasal et un échantillon de salive sont plongés ensemble étant soumis à une incubation à une température comprise entre 60 °C et 100 °C pendant 1 à 25 minutes avant d'être utilisé dans la réaction d'amplification d'acide nucléique, et la réaction d'amplification d'acide nucléique étant réalisée à l'aide du produit de l'incubation, le milieu de transport d'échantillon utilisé dans la réaction d'amplification d'acide nucléique étant de préférence dilué 2 à 10 fois avant d'être appliqué à la réaction d'amplification d'acide nucléique.

9. Procédé selon la revendication 1, l'agent pathogène respiratoire étant un virus respiratoire et/ou une bactérie respiratoire, le virus respiratoire étant de préférence le virus de la grippe, le virus respiratoire syncytial (VRS), l'adénovirus, l'entérovirus, le virus parainfluenza (PIV), le métapneumovirus (MPV), le bocavirus, le rhinovirus et/ou le coronavirus.

10. Procédé selon la revendication 9, le coronavirus étant le coronavirus 2 du syndrome respiratoire aigu sévère (SARS-CoV-2), la composition pour réaction d'amplification d'acide nucléique contenant de préférence des amorces utilisées pour amplifier au moins l'un des gènes RdRP, S et N du SARS-CoV-2.

11. Procédé selon la revendication 1, la réaction d'amplification d'acide nucléique étant une réaction d'amplification d'acide nucléique en temps réel ; ou la composition pour réaction d'amplification d'acide nucléique contenant des amorces, une sonde et une enzyme.

12. Un kit de prélèvement destiné à détecter un agent pathogène respiratoire, ledit kit de prélèvement comprenant :
(a) un écouvillon nasal, (b) un dispositif de prélèvement de salive et (c) un récipient contenant un milieu de transport d'échantillon, le dispositif de prélèvement de salive étant de préférence un écouvillon à salive ; le kit de prélèvement étant un kit d'autoprélèvement ; le milieu de transport d'échantillon étant un tampon de maintien cellulaire ou un tampon d'inactivation cellulaire ; ou, lorsqu'un échantillon prélevé par l'écouvillon nasal et le dispositif de prélèvement de salive est imprégné dans le milieu de transport d'échantillon, le milieu de transport d'échantillon étant utilisé dans la réaction d'amplification d'acide nucléique sans processus d'isolation d'acide nucléique, l'écouvillon de salive étant de préférence appliqué sur une partie supralinguale ou une partie sublinguale ; ou le tampon de maintien cellulaire étant une solution saline ou un tampon à base de solution saline équilibrée.

13. Un kit destiné à la détection d'un agent pathogène respiratoire, ledit kit comprenant :
(a) le kit de prélèvement selon la revendication 12 et
(b) une composition de réaction d'amplification d'acide nucléique en temps réel contenant (i) des amorces pour amplifier une molécule d'acide nucléique de l'agent pathogène respiratoire ; (ii) une sonde s'hybridant avec la molécule d'acide nucléique de l'agent pathogène respiratoire et (iii) une enzyme comprenant une ADN polymérase, l'enzyme comprenant de préférence une Taq polymérase à démarrage à chaud et une transcriptase inverse ; ou l'agent pathogène respiratoire étant un virus respiratoire et/ou une bactérie respiratoire, le virus respiratoire étant de préférence le virus de la grippe, le virus respiratoire syncytial (VRS), l'adénovirus, l'entérovirus, le virus parainfluenza (PIV), le métapneumovirus (MPV), le bocavirus, le rhinovirus et/ou le coronavirus.

14. Le kit selon la revendication 13, le coronavirus étant le coronavirus 2 du syndrome respiratoire aigu sévère (SARS-CoV-2), la molécule d'acide nucléique de l'agent pathogène respiratoire correspondant de préférence à au moins un des gènes suivants : le gène RdRP, le gène S et le gène N du SARS-CoV-2.

15. Procédé de prélèvement destiné à détecter un agent pathogène respiratoire, ledit procédé comprenant :
(a) le prélèvement d'un échantillon par écouvillonnage nasal et d'un échantillon de salive à l'aide du kit de prélèvement selon la revendication 12 ; et
(b) l'imprégnation de l'échantillon par écouvillonnage nasal et de l'échantillon de salive dans un milieu de transport d'échantillons contenu dans un récipient.
